# EUROPEAN PATENT APPLICATION

(11) **EP 1 647 818 A2**
(43) Date of publication of application: **19.04.2006**
(21) Application number: 05022114.2
(22) Date of filing: 11.10.2005
(51) Int. Cl.: G01N 1/30, G01N 33/48, G01N 33/50, G01N 33/574

(54) **Pseudo-tissue for quality control and quality control method using the same**

(30) Priority: 15.10.2004 JP 2004302248; 01.02.2005 JP 2005024918
(71) Applicant: SYSMEX CORPORATION, Kobe-shi, Hyogo 651-0073 (JP)
(72) Inventor: Yamasaki, Masatoshi Sysmex Corporation, Kobe-shi Hyogo 651-0073 (JP); Fujimoto, Keiji Sysmex Corporation, Kobe-shi Hyogo 651-0073 (JP)
(74) Representative: HOFFMANN EITLE

(57) **Abstract**

A pseudo-tissue for quality control is described, which includes a nucleic acid component selected from the group consisting of a nucleic acid and a cell including a nucleic acid, and a gel for holding nucleic acid component.

## Description

### FIELD OF THE INVENTION

The present invention relates to a pseudo-tissue for quality control (hereinafter, simply referred to as pseudo-tissue), and a quality control method using the same.

### BACKGROUND

In recent years, gene examination has rapidly spread in the field of clinical diagnosis. Gene examination means to examine the existence of mutations and karyotype's concerning genetic diseases for the clinical purpose by analyzing nucleic acids, chromosomes, and the like. As an example of gene examination, there is an examination which determines whether or not a nucleic acid, originated from oncocyte, exists within a tissue that has been excised from a living organism. This examination process comprises three primary processes: pre-processing, nucleic acid amplification, and detection.

Pre-processing includes a variety of methods. As an example, first, a reagent for pre-processing is added to a tissue that has been excised from a living organism for the purpose of examination. Next, the tissue to which the reagent has been added is homogenized, and the target nucleic acid is extracted, refined, and collected from the obtained homogenate.

In the nucleic acid amplification, the above described sample containing collected target nucleic acid is contained in a sample container, into which reagents such as an enzyme, a primer, and the like are added so that the target nucleic acid is amplified through the nucleic acid amplification reaction.

In the detection, determination of the existence or calculation of the concentration of the target nucleic acid in the excised tissue is carried out through fluorometry of the target nucleic acid that has been dyed with a fluorescent or through turbidimetry of a by-product that has been produced in proportion to the amplification.

In a gene examination, as described above, a variety of factors in each process (pre-processing, nucleic acid amplification, and detection) affect the results of the measurement. Therefore, it is important to control the quality in order to secure the quality and reliability in the respective processes in the field of gene examination.

Concerning the nucleic acid amplification and the detection, a control solution that contains the target nucleic acid of which the concentration has been known, is used for positive control, and a control solution that contains a nucleic acid that is not to be amplified, is used for negative control, and thus, quality control are carried out. The positive control is to confirm whether or not the amplification of the target nucleic acid and the detection of the amplified target nucleic acid are appropriately carried out, whereas, the negative control is to confirm that the nucleic acid that is not to be amplified is not amplified.

Concerning the pre-processing, a problem may arise where the degree of homogenization in the excised tissue differs depending on the level of skill of the person who carries out the pre-processing, and therefore, quality control of the pre-processing is also important. However, no quality control is carried out on the pre-processing in the current state. In the case where homogenization is manually carried out, the degree of homogenization of the excised tissue differs depending on the level of skill of the person who carries out the pre-processing. In addition, in the case where homogenization is automatically carried out in the automated crushing device, the degree of homogenization of the excised tissue differs depending on the setting of the conditions for homogenization such as the number of revolutions of the blender, wear and tear of the blender due to continuous use, and the like. Accordingly, quality control of the pre-processing such as the control of homogenization conditions is necessary both in the case where homogenization is manually carried out and in the case where homogenization is carried out in the automated crushing device. Therefore, development of a material for quality control and development of a quality control method using this material for quality control are desired in order to control quality of pre-processing.

An object of the present invention is to provide a pseudo-tissue for quality control that can be used in the quality control of pre-processing in a gene examination.

### SUMMARY

The present invention provides a pseudo-tissue for quality control and a quality control method using the pseudo-tissue.

The scope of the present invention is defined solely by the appended claims, and is not affected to any degree by the statements within this summary.

A first aspect of the present invention relates to a pseudo-tissue for quality control. The pseudo-tissue comprises: a nucleic acid component selected from the group consisting of a nucleic acid and a cell including a nucleic acid; and a gel for holding nucleic acid component.

A second aspect of the present invention relates to a method for quality control using a pseudo-tissue comprising the steps of;
measuring the concentration of a nucleic acid in a sample that is obtained by carrying out a nucleic acid extracting process including homogenization on a pseudo-tissue having a predetermined nucleic acid concentration, the pseudo-tissue comprising a nucleic acid component and a support for holding the nucleic acid component, the nucleic acid component selected from the group consisting of a nucleic acid and a cell including a nucleic acid; and
comparing the measured concentration with the predetermined nucleic acid concentration so as to determine whether or not said nucleic acid extracting process was appropriately carried out.

A third aspect of the present invention relates to a method for quality control using a pseudo-tissue comprising the steps of;
amplifying a target nucleic acid in a sample that is obtained by carrying out a nucleic acid extracting process including homogenization on a pseudo-tissue having a predetermined target nucleic acid concentration, the pseudo-tissue comprising a nucleic acid component and a support for holding the nucleic acid component, the nucleic acid component selected from the group consisting of a target nucleic acid and a cell including a target nucleic acid;
measuring the concentration of the target nucleic acid originating from said pseudo-tissue by detecting the amplified target nucleic acid; and
comparing the measured concentration with the predetermined nucleic acid concentration so as to determine whether or not the steps of amplifying and measuring were appropriately carried out.

### BRIEF DESCRIPTION OF THE DRAWINGS

Fig 1 is a diagram showing absorbance determining unit 15 made of a personal computer 13 and a spectrophotometer 14.
Fig 2 is a flow chart showing the flow of the quality control operation.
Fig 3 is a diagram showing a nucleic acid amplification detecting unit.
Fig 4 is a diagram showing the entire configuration of the determining part of the nucleic acid amplification detecting unit shown in Fig 3.
Fig 5 is a flow chart showing the flow of the quality control operation in the nucleic acid amplification detecting unit of Fig 3.
Fig 6 schematically shows the preparation of the samples for measurement that was carried out in Experiment 1.
Fig 7 is a diagram schematically showing the pseudo-tissue.
Fig 8 is a graph showing the concentration of RNA in Table 1
Fig 9 schematically shows the preparation of the samples for measurement that was carried out in Experiment 2.
Fig 10 is a graph showing the concentration of RNA in Table 2.
Fig 11 schematically shows the preparation of the samples for measurement that were carried out in the Experiment 3.
Figs 12(1), (2) and (3) are graphs showing the time to start amplification of samples v, vi and vii for measurement, respectively.

### DESCRIPTION OF THE PREFERRED EMBODIMENTS

"Pseudo-tissue" indicates a pseudo-living organism tissue. A lymph node or the like that has been excised from a living organism is assumed to be an example of a pseudo-tissue. A pseudo-tissue comprises a nucleic acid or cells and a support that can hold the nucleic acid or the cells. The pseudo-tissue is used for the quality control of pre-processing in a gene examination.

"Nucleic acid" in the present embodiment includes artificial nucleic acids such as PNA, BNA, and analogs to these, in addition to DNA and RNA. In addition, a nucleic acid that is encapsulated in a protein or the like such as an armored RNA (US Patent No. 5677124) may be used as a nucleic acid in the present invention. The origin of the nucleic acid is not particularly limited, and the nucleic acid may be extracted from cells or may be artificially synthesized.

"Nucleic acid extracting process" in the present embodiment includes homogenization on the pseudo-tissue and centrifugation after the homogenization. The nucleic acid extracting process may include known nucleic acid extracting method such as a phenol method, an alkaline method, a phenol chloroform method etc.

Cells are not particularly limited, as long as they are cells that contain a nucleic acid, but oncocytes are preferable. In addition, it is preferable for cells to be cells that contain at least one selected from genes that code a portion or the entirety of a material to be examined in the gene examination, mRNA which corresponds to these genes, and a partial sequence of these.

A tumor marker can be cited as the material to be examined in the gene examination. Cytokeratin (CK), carcino-embryonic antigens, α-fetoprotein, tissue polypeptide antigens, immunosuppressive acid protein, α-fetoprotein, basic fetoprotein, PIVKA, DUPAN, elastase, SCC antigens, Pro GRP, neuron specific enolase, urinary NMP-22, prostatic acid phosphatase, γ-seminoprotein and the like can be cited as examples of the tumor marker.

The support in the present invention is a gel, and it is preferable for the support to be solid at room temperature (25 °C), and to be fluidized when heated to a certain temperature. In addition, it is preferable for the support to have the same degree of hardness as the living organism tissue in the solid state.

The gel that is used as the support includes a solvent and a gelling agent. A gelling agent is a material having properties such that it converts a solution to a gel when added to a solvent. Natural polymers, such as agar, agarose, carageenan, alginic acid, alginates, pectin, collagen, gelatin and gluten, as well as synthetic polymers, such as polyvinyl alcohol (PVA), polyethylene glycol (PEG) and polyacrylamide (PAA) , can be cited as examples of a gelling agent. One or more from among these synthetic and natural polymers can be used for a pseudo-tissue in the present embodiment. Though the solvent is not particularly limited, water, TE (tris EDTA), TAE (tris-acetate EDTA) and TBE (tris-borate EDTA), for example, can be used.

Agar is a polysaccharide that is formed of galactose and galactose derivatives, which are included in the cell walls of Rhodophyceae etc. The origin of agar is not particularly limited, as long as it can be used as a gelling agent, and agar that is refined from Gelidium amansii, Gracilaria verrucosa, Grateloupia filicina, Najasmarina, Gigartina tenella, Gelidium subcostatum, and Gelidium japonicum, for example, can be used.

Agarose is a polysaccharide of which the main structure is made of D-galactose and 3, 6-anhydro-L-galactose, which are alternately connected, where the first carbon of D-galactose and the fourth carbon of 3, 6-anhydro-L-galactose are connected through β-glycoside linkage, and the first carbon of 3, 6-anhydro-L-galactose and the third carbon of D-galactose are connected through α-glycoside linkage. The origin of agarose is not particularly limited, as long as it can be used as a gelling agent. Agarose is included in agar, usually with a ratio of approximately 70 %., and agarose with a high purity can be obtained by refining it. A variety of agarose contents in agarose ranging from a low degree of refinement to a high degree of refinement can be utilized.

Carageenan is a polysaccharide that is included in Rhodophyceae etc. The origin of carageenan is not particularly limited, as long as it can be used as a gelling agent. Carageenan that has been refined from living organisms that belong to, for example, Gigartinaceae, Solieriaceae and Hypneaceae can be used. κ-carageenan and λ-carageenan, for example, can be used in the present embodiment.

κ-carageenan has a basic structure where D-galactose-4-sulfuric acid and 3, 6-anhydro-D-galactose are alternately repeated through β-glycoside linkage between the first carbon of D-galactose-4-sulfuric acid and the fourth carbon of 3, 6-anhydro-D-galactose, and through α-glycoside linkage between the first carbon of 3, 6-anhydro-D-galactose and the third carbon of D-galactose-4-sulfuric acid.

λ-carageenan has a basic structure where D-galactose and D-galactose-2, 6-disulfuric acid are alternately repeated through β-glycoside linkage between the first carbon of D-galactose and the fourth carbon of D-galactose-2, 6-disulfuric acid, and through α-glycoside linkage between the first carbon of D-galactose-2, 6-disulfuric acid and the third carbon of D-galactose.

Alginic acid is a polysaccharide that is contained in the cell walls of Phaeophyceae etc. Alginic acid has a chain structure where D-mannuronic acid with β-1, 4 linkage and L-guluronic acid with α-1, 4 linkage are connected. The origin of alginic acid is not particularly limited, as long as it can be used as a gelling agent, and alginic acid or alginate (sodium alginate, calcium alginate, magnesium alginate, ammonium alginate and the like), for example, can be used.

Pectin is a polysaccharide that is contained in the cell walls of plants etc. the main component of pectin is an acidic polysaccharide which has a main chain, where L-rhamnose with α-1, 2 linkage is partially mixed in with D-galacturonic acid with α-1, 4 linkage, and has a side chain that is rich in neutral saccharides, such as arabinose and galactose, that is to say, rhamnogalacturonan-I. The origin of pectin is not particularly limited, as long as it can be used as a gelling agent.

Collagen is a protein that becomes the main component of a matrix on the outside of animal cells. Collagen has a triple spiral structure made of three polypeptide chains. In addition, gelatin is a type of derivative protein obtained by treating collagen with hot water. The origin of collagen and gelatin is not particularly limited, as long as they can be used as a gelling agent, and collagen and gelatin that has been refined from animal tissue or artificially synthesized can be used in the present embodiment.

Gluten is a viscous protein that is included in crops etc. Gluten is formed through association between a glutelin based protein and a prolamin based protein. The origin of gluten is not particularly limited, as long as it can be used as a gelling agent. For instance, gluten which has been extracted from wheat or barley, or artificially synthesized can be used in the present embodiment.

PVA is obtained by hydrolyzing polyvinyl acetate, and is a synthesized polymer of which the main component is vinyl alcohol units.

PEG is obtained by anion polymerizing ethylene oxide with alkaline, or by cation polymerizing ethylene oxide through proton initialization, and is a synthesized polymer of which the main component is ethylene glycol units.

PAA is obtained by polymerizing a vinyl group of acrylamide or by hydrogen transfer polymerizing an amide group, and is a synthesized polymer of which the main component is acrylamide units.

The degree of polymerization of PVA, PEG and PAA, which are used in the present embodiment, is not particularly limited, as long as these polymers can be used as a gelling agent.

Quality control of pre-processing in gene examination is carried out through measurement of absorbance (optical density), as well as nucleic acid amplification, nucleic acid detection and the like, using the above described pseudo-tissue.

In the following, quality control of pre-processing in the measurement of absorbance (Example 1) and quality control of pre-processing in nucleic acid amplification and nucleic acid detection (Examples 2 and 3) are described.

### (Example 1)

Quality control of pre-processing through measurement of absorbance includes the step of measuring the concentration of nucleic acid in a sample that has been obtained by carrying out a nucleic acid extracting process, including homogenization, on a pseudo-tissue for quality control, which is made of a nucleic acid or cells, and a support that can hold the nucleic acid or the cells, and has a predetermined target value, as well as the step of comparing the measured value of the concentration of nucleic acid with the target value, so as to determine whether or not the above described nucleic acid extracting process has been appropriately carried out.

### <Calculation of Target value α>

First, the target value is calculated.

The target value is the measured value of the concentration of the nucleic acid that has been calculated by carrying out predetermined pre-processing on a pseudo-tissue that has been prepared in accordance with a set protocol and determining the absorbance. This target value is compared with the measured value of the concentration of the nucleic acid of the object to be measured, and thereby, quality control of the pre-processing that has been carried out on the object to be measured is carried out.

In the following, preparation of a pseudo-tissue α for calculating the target value is described.

Prior to the measurement of absorbance of pseudo-tissue α, a nucleic acid solution that includes a nucleic acid, or a cell suspension that includes cells and a reagent that includes a buffer (hereinafter referred to as homogenizing reagent) are mixed, and this mixture is referred to as reference sample α. In the case where a nucleic acid solution is used, the concentration of the nucleic acid in reference sample α is calculated by determining the absorbance of this reference sample α, and this is referred to as reference value α. In the case where the cell suspension is used, the mixture of the cell suspension and the homogenizing reagent is sufficiently homogenized, and this is referred to as reference sample α. The concentration of the nucleic acid is calculated by determining the absorbance of reference sample α, and this is referred to as reference value α. Here, the number of cells in the cell suspension is counted in advance using a cell counting board (for example, counting chamber 8100105, made by AS ONE CORPORATION) or a cell number counting device (for example, particle number counting analyzer CDA-500, made by Sysmex Corporation).

Next, pseudo-tissue α is prepared, using the same amount of the same nucleic acid solution or cell suspension that is used for the preparation of reference sample α.

At the time of the preparation of pseudo-tissue α, first, a solvent is contained in a container such as a micro-tube.

A gelling agent is added to the solvent in the container, and the mixture of the gelling agent and the solvent, as well as the container that contains this mixture, are heated in a thermostatic bath or a microwave oven, so that the gelling agent is completely solved in the solvent, and thus, a gelling agent solution is prepared.

The container that contains the gelling agent solution is left at room temperature, or in a cool place, for example, within a refrigerator, and thereby, the gelling agent solution is cooled.

When the gelling agent solution becomes of a temperature that is no higher than a certain temperature, it solidifies, so as to be a solid in gel form. Before it becomes solid, the same amount of the same nucleic acid solution or cell suspension as that used at the time of the preparation of reference sample α is added to the gelling agent solution using a pipet. Next, the mixture of the gelling agent solution and the nucleic acid solution or cell suspension is mixed, so that the nucleic acid or the cells are not unevenly distributed in the mixture.

Furthermore, when the temperature of the liquid is lowered until the gelling agent solution becomes a solid in gel form, the gelling agent solution that contains the nucleic acid or the cells is converted to a gel and becomes pseudo-tissue α made up of the nucleic acid or the cells, and a support that holds the nucleic acid or the cells.

The same process as the pre-processing in gene examination is carried out on pseudo-tissue α that has been prepared as described above, and the absorbance is determined.

In the pre-processing, first, a homogenizing reagent is added to pseudo-tissue α. The added amount of homogenizing reagent is adjusted so that the volume of the mixture of pseudo-tissue α and the homogenizing reagent becomes equal to the volume of reference sample α.

Next, the mixture of pseudo-tissue α and the homogenizing reagent is homogenized, and a homogenate is prepared.

The homogenate that is obtained from pseudo-tissue α is centrifuged, and the supernatant above the sediment is contained in another container. The absorbance of this clear fluid in 260 nm is determined, and then, the concentration of the nucleic acid is measured.

In the case where the concentration of the nucleic acid in the clear fluid that is obtained from pseudo-tissue α is equal to or in the proximity of reference value α (for example, in the case where this concentration of the nucleic acid indicates the value of 70 % or higher, preferably 80 % or higher, of reference value α), it can be assumed that the nucleic acid that is included in the clear fluid of the homogenate of pseudo-tissue α can be effectively extracted through homogenization, and this measured value of the concentration of the nucleic acid can be used as target value α.

### <Quality control of Pre-processing through Measurement of absorbance>

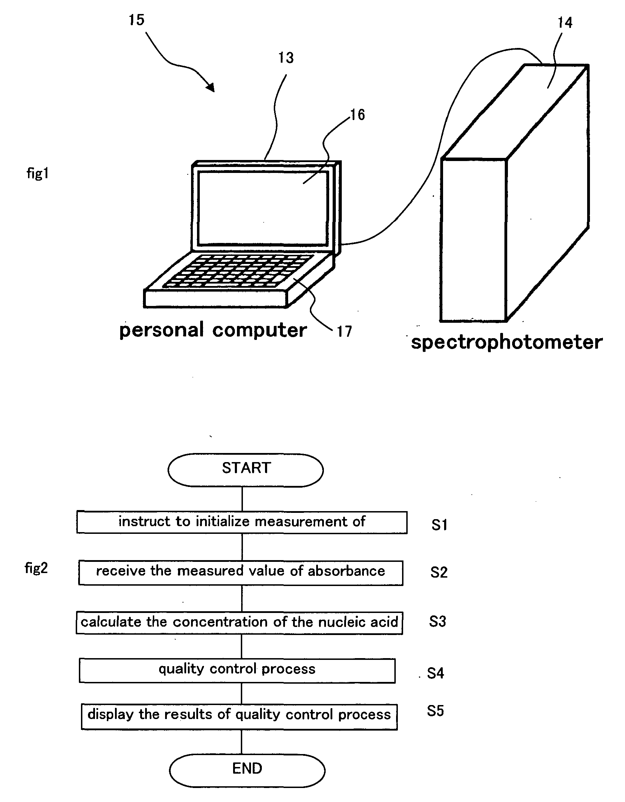

In the following, the configuration of an absorbance determining unit 15 is described in reference to Fig 1.

Fig 1 is a diagram showing absorbance determining unit 15 made of a personal computer 13 and a spectrophotometer 14. Personal computer 13 and spectrophotometer 14 are connected so that data communication is possible.

Personal computer 13 is provided with a display part 16, an input part 17 for inputting information or for operation, and a control part having a CPU, a ROM, a RAM and the like. In addition, a computer program for remotely operating spectrophotometer 14 and for a quality control process is installed in the hard disk of personal computer 13. In addition, the CPU in the control part executes the above described computer program, and thereby, the below described quality control operation is carried out.

Spectrophotometer 14 is provided with a light emitting element, such as an LED, or a laser emitting part and a light receiving element, such as a photodiode or a photomultiplier, and it becomes possible to set a specimen between the light emitting element and the light receiving element. Light that has been radiated from the light emitting element and has passed through the specimen is detected by the light receiving element in this spectrophotometer 14, and thereby, it is possible to determine the absorbance of the specimen. In addition, it is possible to remotely operate spectrophotometer 14 from personal computer 13, and the initiation of the determination operation can be instructed from personal computer 13.

Next, the quality control operation using absorbance determining unit 15 is described in reference to Fig 2. Fig 2 is a flow chart showing the flow of the quality control operation.

A pseudo-tissue β of the same lot as pseudo-tissue α is used for the quality control.

Pre-processing, such as homogenization, centrifugation and collection of the supernatant above the sediment, is carried out on pseudo-tissue β, in the same manner as the pre-processing that is carried out on pseudo-tissue α.

Absorbance determining unit 15 carries out a quality control operation on the basis of target value α, as described below, using the supernatant that has been obtained by carrying out pre-processing on pseudo-tissue β.

First, the operator sets the supernatant of pseudo-tissue β in spectrophotometer 14.

Next, the operator inputs attribute information (target value or the like) of pseudo-tissue β using input part 17, and an instruction to initialize determination is inputted by carrying out a predetermined operation, such as, for example, clicking a determination initialization button that is displayed on the screen. When the inputted attribute information is stored in the RAM, and the input of the instruction to initialize determination is accepted, the control part of personal computer 13 instructs spectrophotometer 14 to initialize measurement of absorbance in 260 nm of the supernatant (S1) . This instruction makes spectrophotometer 14 carry out measurement of absorbance of the supernatant.

The control part of personal computer 13 receives the measured value of absorbance by spectrophotometer 14 (S2), and calculates the concentration of nucleic acid on the basis of the received measured value of absorbance (S3).

Next, the control part of personal computer 13 compares the calculated concentration of nucleic acid with target value α, and thereby, carries out a quality control process (S4). Concretely speaking, the control part of personal computer 13 determines that the pre-processing that has been carried out on pseudo-tissue β was appropriate in the case where the concentration of nucleic acid is in a predetermined range (for example, 70 % or more of target value α, preferably 80 % or more), and determines that the preprocessing that has been carried out on pseudo-tissue β was inappropriate in the case where the concentration of nucleic acid is outside the predetermined range (for example, less than 70 % of target value α, preferably less than 80 %).

Then, the control part, of personal computer 13 displays the results of the quality control process in S4 on display part 16 (S5) .

Here, though spectrophotometer 14 automatically transmits the results of measurement of absorbance to personal computer 13 according to the present embodiment, the results of determination may be displayed on spectrophotometer 14, and the displayed results of determination may be manually inputted into personal computer 13.

In addition, though spectrophotometer 14 automatically transmits the results of determination to personal computer 13 according to the present embodiment, the results of determination may be displayed on spectrophotometer 14, and the displayed results of determination may be compared with target value α without using personal computer 13.

### (Example 2)

Quality control of the pre-processing through nucleic acid amplification and nucleic acid detection includes: the amplification step of amplifying the target nucleic acid that is included in the sample obtained by homogenizing a pseudo-tissue for quality control which is made of the target nucleic acid or cells that include the target nucleic acid and a support that can hold the target nucleic acid or the cells that include the target nucleic acid, and has a predetermined target value; the measuring step of detecting the amplified target nucleic acid and measuring the concentration of the target nucleic acid originating from the pseudo-tissue; and the step of comparing the measured value of the concentration of the target nucleic acid of the pseudo-tissue with the target value so as to determine whether or not homogenization, the amplification step and the measuring step were appropriately carried out.

### <Calculation of Target value γ>

In the following, the preparation of a pseudo-tissue γ for the calculation of the target value is described.

Prior to the nucleic acid amplification and the nucleic acid detection of pseudo-tissue γ, first, a cell suspension that contains the target nucleic acid or cells that include the target nucleic acid, and a homogenizing reagent are mixed so as to prepare a reference sample γ. In the case where a solution contains cells, a sufficiently homogenized mixture of a cell suspension fluid and a homogenizing reagent is used as reference sample γ. Here, the number of cells in the cell suspension is counted in advance.

Next, the same amount of the same solution that contains the target nucleic acid or the cells as that used for the preparation of reference sample γ is used to prepare pseudo-tissue γ.

Pseudo-tissue γ is prepared in the same manner as the preparation of the above described pseudo-tissue α.

A homogenizing reagent is added to pseudo-tissue γ. The added amount of homogenizing reagent is adjusted so that the volume of the mixture of pseudo-tissue γ and the homogenizing reagent becomes equal to the volume of reference sample γ.

The mixture of pseudo-tissue γ and the homogenizing reagent is homogenized, so that a homogenate is prepared.

The homogenate that has been obtained from pseudo-tissue γ is centrifuged, and a supernatant above the sediment is contained in another container. In the following, this supernatant is referred to as sample γ for measurement.

Nucleic acid amplification and nucleic acid detection are carried out on the above described reference sample γ and sample γ for measurement.

In the nucleic acid amplification, an enzyme reagent is added to supernatants above the sediments of reference sample γ and sample γ for measurement, and the target nucleic acid is amplified in accordance with a non-nucleic acid amplification method (in the case where the target nucleic acid is RNA, cDNA that corresponds to the base sequence of this RNA is amplified).

Any known nucleic acid detection method can be used for the detection.

In the case where an insoluble material, such as magnesium pyrophosphate is refined at the same time as the nucleic acid amplification, the turbidity of the supernatant above the sediment in the reactive liquid is visually inspected, the turbidity is measured through the measurement of absorbance or the intensity of the scattered light of the reactive liquid, or the reactive liquid is filtered through a colored filter so that the residue on the filter can be inspected, and thereby, the target nucleic acid can be detected (Pamphlet of International Publication WO 01/83817).

In addition, in the case where the nucleic acid amplification is carried out under the existence of a fluoro-chrome, which is a double strand intercalator, such as ethidium bromide, SYBR GREEN I or Pico Green, the target nucleic acid detection can be carried out through the measurement of fluorescent light of the reactive liquid.

In the measurement of the turbidity or in the measurement of the fluorescent light, an increase in the turbidity or in the intensity of fluorescent light can be observed together with an increase in the product. If this increase is monitored in real time, an increase in the amount of nucleic acid and an increase in the turbidity or in the intensity of fluorescent light can be traced at the same time in a closed system.

In the case where the nucleic acid detection is carried out through visual inspection, the turbidity of reference sample γ and the turbidity of sample γ for measurement are compared, and thereby, whether or not pre-processing was precisely carried out on sample γ for measurement can be confirmed.

In the case where the nucleic acid detection is carried out by measuring turbidity, the concentration of the target nucleic acid of sample γ for measurement and the concentration of the target nucleic acid of reference sample γ (hereinafter referred to as reference value γ) are compared, in the case where these show equal values or close values (for example, in the case where the concentration of the target nucleic acid of sample γ for measurement shows a value that is 70 % or more of reference value γ, preferably 80 % or more), it can be assumed that the target nucleic acid that is contained in sample γ for measurement originating from pseudo-tissue γ was effectively extracted through homogenization, and this value obtained by measuring the concentration of the target nucleic acid can be referred to as target value γ. In addition, in the case where an increase in the turbidity or in the intensity of fluorescent light is monitored in real time, the time required for the nucleic acid that is contained in sample γ for measurement to start being amplified (amplification starting time) and the amplification starting time of reference sample gamma (hereinafter referred to as reference value Υt) are compared. In the case where these show equal or close values, it can be assumed that the target nucleic acid that is contained in sample for measurement γ was effectively extracted through homogenization, and the starting time thereof can be referred to as target value γt.

### <Quality control of Pre-processing through Nucleic Acid Amplification and Nucleic Acid Detection>

Next, a quality control method on the basis of target value Y or γt is described.

A pseudo-tissue δ is prepared for quality control. Pseudo-tissue δ is a pseudo-tissue of the same lot as the above described pseudo-tissue Y.

Pre-processing, such as homogenization, centrifugation and collection of the supernatant above the sediment is carried out on pseudo-tissue δ, in the same manner as the pre-processing that has been carried out on pseudo-tissue δ, and thereby, sample for measurement δ is prepared.

The same operations for the nucleic acid amplification and the nucleic acid detection that have been carried out on the above described sample for measurement γ are carried out on sample for measurement δ, and the concentration of the target nucleic acid and/or the amplification starting time of sample for measurement δ is measured.

In the case where the value obtained by measuring the concentration of the target nucleic acid or the amplification starting time of sample for measurement 5 is equal or close to the above described target value γ or γt, it can be confirmed that the process that has been carried out pseudo-tissue δ was appropriate.

Here, at least one process from among the above described pre-processing, the nucleic acid amplification and the nucleic acid detection may be automatically carried out by a unit.

A nucleic acid amplification detecting unit, for example, can be cited as a unit that automatically carries out nucleic acid amplification or nucleic acid detection.

GD-100 (made by Sysmex Corporation) which helps in the diagnosis of cancer metastasis conducted on excised tissue during a cancer operation can be cited as an example of a nucleic acid amplification detecting unit. This unit allows mRNA within the excised tissue to be used as a template for the reverse transcription from mRNA to cDNA in accordance with an RT-LAMP (reverse transcription loop mediated isothermal amplification, Eiken Chemical Co., Ltd.) method, and this cDNA is amplified and the turbidity of the solution which increases together with the amplification measured, and thereby, the starting time of the amplification and the number of copies of mRNA within the excised tissue can be calculated in the unit.

This nucleic acid detecting unit allows the content of nucleic acid from a cancer marker (for example, cytokeratin 19) in the tissue that has been excised from a living organism to be quantified.

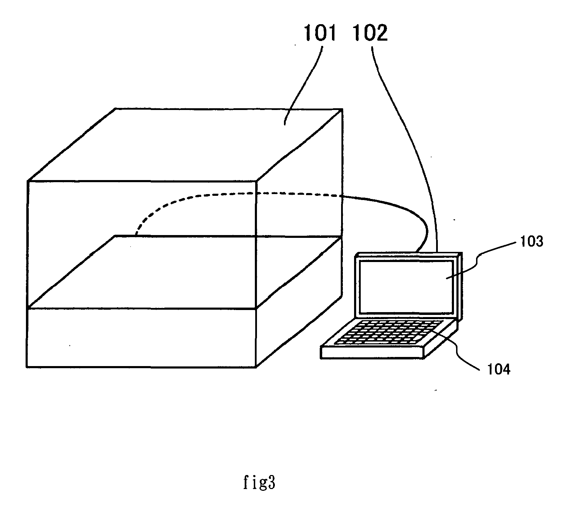

Fig 3 is a diagram showing a nucleic acid amplification detecting unit.

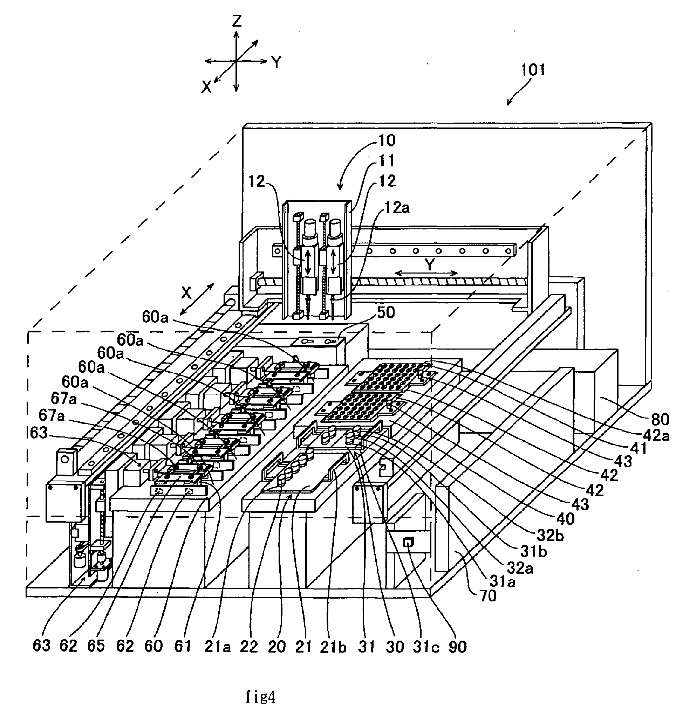

Fig 4 is a diagram showing the entire configuration of the determining part of the nucleic acid amplification detecting unit shown in Fig 3.

First, the nucleic acid amplification detecting unit is described in reference to Fig 3. The nucleic acid amplification detecting unit is formed of a determining part 101 and a personal computer 102 for data processing that is connected to determining part 101 through a communication line.

The configuration of personal computer 102 is the same as the configuration of personal computer 13 described in Example 1. Personal computer 102 is provided with a display part 103, an input part 104 for inputting information or for operation, and a control part having a CPU, a ROM, a RAM and the like.

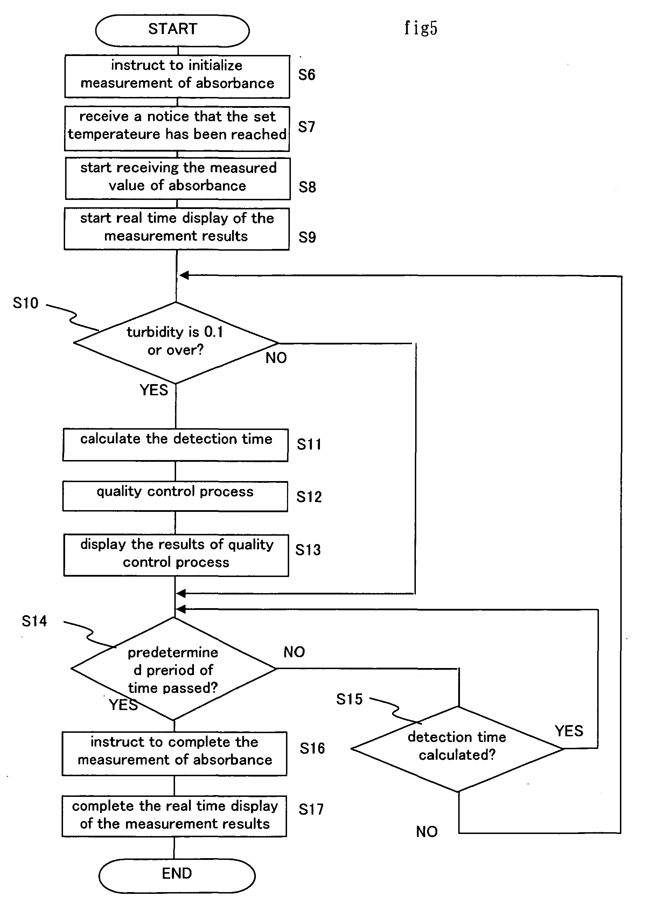

As shown in Fig 5, determining part 101 includes a delivering mechanism 10, a sample container setting part 20, a reagent container setting part 30, a chip setting part 40, a chip disposal part 50 and a reaction detecting part 60 made of five reaction detecting blocks 60a.

As shown in Fig 5, determining part 101 controls the unit by means of a microcomputer, and incorporates a control part 70 for controlling input into/output from the outside of the unit, and a power supply part 80 for supplying power to the entirety of the unit, including control part 70. In addition, an emergency shutdown switch 90 is placed at a predetermined point in front of determining part 101.

Sample container setting part 20, reagent container setting part 30 and chip setting part 40 are placed in the direction of the X axis. In addition, sample container setting part 20 is placed on the front side of the unit, and reagent container setting part 30 is placed on the rear side of the unit. In addition, five reaction detecting blocks 60a and chip disposal part 50 are placed in the direction of the X axis at points that are at a predetermined distance from sample container setting part 20, reagent container setting part 30 and chip setting part 40 in the direction of the Y axis. That is to say, sample container setting part 20, reagent container setting part 30, chip setting part 40, chip disposal part 50 and the five reaction detecting blocks 60a are arranged in square form (rectangular form).

In addition, delivering mechanism 10 includes an arm part 11 which is moveable in the direction of the X and Y axes (on a plane), and two syringe parts 12 which are independently moveable in the direction of the Z axis (vertically) relative to arm part 11. The two syringe parts 12 are respectively provided with a nozzle part 12a, at the end of which a pipet chip can be mounted.

In addition, as shown in Fig 5, a sample container setting base 21 having five sample container setting holes 21a and handles 21b is engaged in a recess of sample container setting part 20, in such a manner as to be removable. The five sample container setting holes 21a in sample container setting base 21 are provided at predetermined intervals in single column form in the direction of the X axis. Sample containers 22 that contain samples for measurement that have been prepared by pre-processing excised tissue and/or pseudo-tissue are set in the five sample container setting holes 21a of this sample container setting base 21.

A reagent container setting base 31 having two primer reagent container setting holes 31a, two enzyme reagent container setting holes 31b and handles 31c is engaged in a recess of reagent container setting part 30, in such a manner as to be removable. The two primer reagent container setting holes 31a and the two enzyme reagent setting holes 31b of reagent container setting base 31 are respectively provided at a predetermined distance in the direction of the Y axis. Two primer reagent containers 32a, which contain two types of primer reagents, and two enzyme reagent containers 32b, which contain enzyme reagents that correspond to these two types of primer reagents, are set in primer reagent container setting holes 31a and enzyme reagent setting holes 31b of this reagent container setting base 31, respectively. Here, primer reagent container 32a which contains a primer reagent that corresponds to mRNA of cytokeratin 19 (CK19) and enzyme reagent container 32b which contains an enzyme reagent of CK19 mRNA are placed in primer reagent container setting hole 31a and enzyme reagent container setting hole 31b on the left side when viewed from the front. In addition, primer reagent container 32a which contains a primer reagent of mRNA of β actin as an internal reference material, and enzyme reagent container 32b which contains an enzyme reagent of β actin mRNA are placed in primer reagent container setting hole 31a and enzyme reagent container setting hole 31b on the right side when viewed from the front.

Here, the internal reference material is not limited to β actin, and mRNA of other housekeeping genes and the like can also be used (Pamphlet of International Publication WO 03/070935).

Two racks 42 having containing holes 42 where thirty-six pipet chips 41 can be contained are respectively engaged in two recesses of chip setting part 40, in such a manner as to be removable. In addition, two removing buttons 43 are provided to chip setting part 40. Racks 42 become of a removable state by pressing these removing buttons 43.

As shown in Fig 5, each reaction detecting block 60a of reaction detecting part 60 is formed of a reaction part 61, two turbidity detecting parts 62 and a lid closing mechanism 63. Each reaction part 61 is provided with a detection cell 65. Lid closing mechanism 63 has

a function of mounting a cell part 67a in detection cell 65.

### (Example 3)

In example 3, the procedure of the operation and the operation of the unit in the case where nucleic acid detection is carried out by means of GD-100 (made by Sysmex Corporation) are concretely described. Here, a case where a pseudo-tissue made of cells and a support which holds the cells is used is described.

### <Calculation of Target value εt >

Prior to the nucleic acid amplification and the nucleic acid detection of the pseudo-tissue, first, a cell suspension that contains cells which are considered to include CK19 mRNA and a homogenizing reagent are mixed so as to prepare a sample, which is then sufficiently homogenized, and this is referred to as reference sample ε. Here, the number of cells in the cell suspension is counted in advance.

Next, the same amount of the same cell suspension as that which is used for the manufacture of reference sample ε, and a pseudo-tissue ε are prepared, in the same manner as the preparation of the above described pseudo-tissue α.

A homogenizing reagent is added to pseudo-tissue ε. The added amount of homogenizing reagent is adjusted so that the volume of the mixture of pseudo-tissue ε and the homogenizing reagent becomes equal to the volume of reference sample ε.

The mixture of pseudo-tissue ε and the homogenizing reagent is homogenized so as to prepare a homogenate.

The homogenate that is obtained from pseudo-tissue ε is centrifuged, and the supernatant above the sediment is contained in another container. Hereinafter, this supernatant is referred to as sample ε for measurement.

Containers which contain reference sample ε and sample ε for measurement are respectively set in sample container setting holes 21a of sample container setting base 21.

In addition, a primer reagent container 32a which contains the primer reagent of CK19 mRNA, and an enzyme reagent container 32b which contains the enzyme reagent of CK19 mRNA are set in primer reagent container setting hole 31a and enzyme reagent container setting hole 31b, on the left side as viewed from the front.

In addition, a primer reagent container 32a which contains the primer reagent of β actin mRNA, and an enzyme reagent container 32b which contains the enzyme reagent of β actin mRNA are set in primer reagent container setting hole 31a and enzyme reagent container setting hole 31b, on the right side as viewed from the front.

In addition, two racks 42, each of which contains thirty-six disposable pipet chips 41, are engaged in the recesses of chip setting part 40. Furthermore, two cell parts 67a of detection cells 65 are set in two detection cell setting holes 61a of the reaction part 61 of each reaction detecting block 60a

Next, the operation of determining part 101 is started. When the operation of determining part 101 is started, arm part 11 of delivering mechanism 10 is moved to chip setting part 40, and two syringe parts 12 move in the downward direction, and thereby, pipet chips 41 are automatically mounted on the ends of nozzle parts 12a. Then, after the two syringe parts 12 have moved upwards, arm part 11 of delivering mechanism 10 is moved in the direction of the X axis, toward a point above the two primer reagent containers 32a which contain the primer reagents of CK19 mRNA and β actin mRNA, and which are set in sample container setting support 31.Then, the two syringe parts 12 are moved in the downward direction, and thereby, the ends of the respective pipet chips 41 are inserted in through the liquid surface of the primer reagents of CK19 mRNA and β actin mRNA within the two primer reagent containers 32a, and the respective primer reagents are drawn into pipet chips 41.

After the primer reagents have been drawn into the pipet chips, the two syringe parts 12 are moved upward, and then, arm part 11 of delivering mechanism 10 is shifted to a point above a reaction detecting block 60a. Then, the two syringe parts 12 are shifted in the downward direction, and thereby, two pipet chips 41, which are mounted on nozzle parts 12a of the two syringe parts 12 are respectively inserted into cell parts 67a of detection cells 65, and the primer reagents of CK19 mRNA and β actin mRNA are respectively discharged into the two cell parts 67a.

After the discharge of the primer reagents, the two syringe parts 12 are moved upward, and arm part 11 of delivering mechanism 10 is moved in the direction of the X axis toward a point above chip disposal part 50. In chip disposal part 50, pipet chips 41 are disposed.

Next, arm part 11 of delivering mechanism 10 is again moved to chip setting part 40, and the same operation as described above is carried out in chip setting part 40, and thereby, two new pipet chips 41 are automatically mounted on the ends of nozzle parts 12a of the two syringe parts 12. Then, arm part 11 of delivering mechanism 10 is moved in the direction of the X axis toward a point above the two enzyme reagent containers 32b which respectively contain the two enzyme reagents of CK19 mRNA and β actin mRNA, and which are set in reagent container setting base 31. After that, the two syringe parts 12 are moved in the downward direction, and thereby, the two enzyme reagents of CK19 mRNA and β actin mRNA within the two enzyme reagent containers 32b are drawn into the syringe parts 12, which are then moved in the upward direction. Then, arm part 11 of delivering mechanism 10 is moved to a point above a reaction detecting block 60a, and after that, the two enzyme reagents of CK19 mRNA and β actin mRNA are discharged into the two cell parts 67a of detection cells 65, respectively. After the discharge of the enzyme reagents, arm part 11 of delivering mechanism 10 is moved to a point above chip disposal part 50, and then, pipet chips 41 are disposed.

Next, arm part 11 of delivering mechanism 10 is again moved to chip setting part 40, and after that, two new pipet chips 41 are automatically mounted on the ends of nozzle parts 12a of the two syringe parts 12. Then, arm part 11 of delivering mechanism 10 is moved in the direction of the X axis toward a point above the two sample containers 22 which respectively contain reference sample ε and sample ε for determination that are set in sample container setting base 21, and after that, reference sample ε and sample ε for measurement are respectively drawn into pipet chips 41.

When reference sample ε and sample ε for measurement are respectively discharged into two cell parts 67a of detection cells 65, the mixtures within the two cell parts 67a are mixed through the automated pipetting of the two syringe parts 12. After this, arm part 11 of delivering mechanism 10 is moved to a point above chip disposal part 50, and then, pipet chips 41 are disposed.

After the respective reagents, reference sample ε and sample ε for measurement have been respectively contained within the above described cell parts 67a, and the lids of cell parts 67a of detection cells 65 have been closed by means of lid closing mechanism 63, the heater of determining part 101 starts operating, so as to heat the liquids within detection cells 65 to approximately 65 °C, and thereby, cDNA is amplified using CK19 mRNA as a template, by means of the above described RT-LAMP. Then, a white, cloudy liquid of magnesium pyrophosphate that is created together with the amplification is monitored in real time through measurement of turbidity. Concretely speaking, cell parts 67a of detection cells 65 are irradiated with light from an LED light source (not shown) at the time of the amplification reaction. The light that has been irradiated is received by a light receiving element, and thereby, the turbidity of the liquid within cell parts 67a of detection cells 65 at the time of the amplification reaction is monitored in real time, and thus, the time when the amplification of CK19 mRNA which is included in reference sample ε and sample ε for measurement starts is calculated. Here, the time when the turbidity reaches 0.1 is calculated as the time when the amplification starts.

The time when the amplification of reference sample ε starts, which is calculated through measurement of turbidity, is referred to as reference value εt. In the case where the time when the amplification of CK19 mRNA of sample ε for measurement starts is the same as or close to εt, it can be assumed that CK19 mRNA that is contained in sample ε for measurement can be effectively extracted through homogenization, and this time when the amplification of CK19 mRNA starts can be referred to as target value εt.

### <Quality control of Pre-processing through Nucleic Acid Amplification and Nucleic Acid Detection>

In the following, the quality control operation is described in reference to Fig 5. Fig 5 is a flow chart showing the flow of the quality control operation in the nucleic acid amplification detecting unit of Fig 3.

A pseudo-tissue η of the same lot as pseudo-tissue ε is used for quality control.

Pre-processing, such as homogenization, centrifugation and collection of the supernatant above the sediment is carried out on pseudo-tissue η, in the same manner as the pre-processing that is carried out on pseudo-tissue ε.

The nucleic acid amplification detecting unit carries out the quality control operation on the basis of target value εt, as described below, using the supernatant above the sediment of pseudo-tissue n.

First, the operator sets the supernatant of pseudo-tissue η in determining part 101.

Next, the operator inputs attribute information (such as the target value) of pseudo-tissue η using input part 104, and inputs the instruction to start measurement, through a predetermined operation, such as for example, clicking a button to start measuring which is displayed on the screen.

The control part of personal computer 102 stores the input attribute information in the RAM, and instructs determining part 101 to start measurement when an input by the operator instructing the determining part to start measuring is accepted (S6) . Determining part 101 first adds a primer, an enzyme or the like to the supernatant, as described above, upon reception of the instruction to start measuring from personal computer 102. When the addition is completed, determining part 101 starts measuring the turbidity of the supernatant, and operates the heater so as to heat the supernatant.

Determining part 101 measures the temperature of the heater and transmits a notice that the set temperature has been reached to personal computer 102 when the temperature of the heater has reached a predetermined temperature (for example, 65 °C), and the control part of personal computer 102 receives this (S7).

Determining part 101 measures the turbidity of the supernatant at constant time intervals (for example, every 5 seconds) and transmits the measured value of the turbidity to personal computer 102 after the transmission of the notice that the set temperature has been reached. The control part of personal computer 102 starts receiving the measured value of the turbidity (S8), and receives every measured value until the completion of the measurement of the turbidity. The control part of personal computer 102 displays the measured value of the turbidity on display part 103 in real time until the completion of the measurement of the turbidity (S9).

Next, the control part of personal computer 102 determines whether or not the measured value of the turbidity becomes 0.1 or higher (S10). In the case where the measured value of the turbidity is less than 0.1, the control part of personal computer 102 carries out the below described process of S14. In the case where the measured value of the turbidity is 0.1 or higher, the control part of personal computer 102 calculates the time from the reception of the notice that the set temperature has been reached to when the turbidity has become 0.1 or higher, and this is referred to as the time to start amplification of the supernatant of pseudo-tissue η (S11).

Next, personal computer 102 carries out a quality control process on the basis of target value εt, using the calculated time to start amplification (S12). Concretely speaking, the time to start amplification and target value εt are compared, an din the case where the time to start amplification is within a predetermined range (for example, target value εt + 0 minutes or higher and less than 5 minutes), it is determined that the homogenization, the nucleic acid amplification and the nucleic acid detection which were carried out on pseudo-tissue η were appropriate. In the case where the time to start amplification is outside the predetermined range (for example, target value εt + 5 minutes or higher), it is determined that the homogenization, the nucleic acid amplification and the nucleic acid detection which were carried out on pseudo-tissue η were inappropriate.

The control part of personal computer 102 displays the result of the quality control process in S11 on display part 103 (S13).

Next, the control part of personal computer 102 determines whether or not a predetermined period of time (for example, 60 minutes) has passed since the start of measurement (S14).

In the case where the predetermined time has not passed, the control part of personal computer 102 determines whether or not the time to start amplification has been calculated (S15). In the case where it has been calculated, the process of S14 is carried out, while in the case where it has not been calculated, the process of S10 is carried out.

Meanwhile, in the case where the predetermined period of time has passed in S14, the control part of personal computer 102 instructs determining part 101 to complete the measurement of the turbidity (S16) . Determining part 101 completes the turbidity measuring operation and turns off the heater upon receiving this instruction to complete the measurement of the turbidity.

Upon the completion of the measurement of the turbidity, the control part of personal computer 102 completes the real time display of the measurement results on display part 103 (S17).

Here, though in the present embodiment, determining part 101 automatically transmits the measurement results to personal computer 102, the measurement results may be displayed on determining part 101, and the displayed measurement results may be manually inputted into personal computer 102.

Here, though in the present embodiment, determining part 101 automatically transmits the measurement results to personal computer 102, the measurement results may be displayed on determining part 101, and the displayed measurement results may be compared with target value εt without using personal computer 102.

Here, in Examples 1 to 3, homogenization of the reference samples and pseudo-tissues may be manually carried out using a pestle or the like. In addition, a cell crushing device such as that described in Japanese Examined Patent Publication H6 (1994) -36732, or an automated crushing device such as that described in Japanese Unexamined Patent Publication 2004-209322 may be used. In the case where homogenization is manually carried out, the precision of the cell crushing operation using a pestle or the like is controlled with the pseudo-tissues of the examples. In the case where homogenization is carried out by means of a device, setting of the conditions for homogenization is controlled, and the state of wear of the blender is checked.

Here, in Examples 1 to 3, a nucleic acid may be extracted from the homogenate in accordance with a known nucleic acid extracting method and refined prior to centrifugation. As for a concrete example of a nucleic acid extracting method, a method where the gene containing bodies are decomposed by means of an enzyme, a surfactant, a chaotropic agent or the like, and after that, a nucleic acid is extracted from the decompose of gene containing bodies in accordance with a phenol method, an alkaline method, a phenol chloroform method or the like is conventionally utilized. Recently, an ion exchanging resin, a glass filter, glass beads, a reagent having protein aggregating effects and the like have been utilized in the process of nucleic acid extraction. In addition, as for a system where a carrier for combining a nucleic acid is used for nucleic acid extraction, a method using glass particles and sodium iodide, a method using hydroxyapatite and the like are known.

### (Experiment 1)

In Experiment 1, a pseudo-tissue made of a nucleic acid and a support that holds the nucleic acid was prepared, and a predetermined process, such as homogenization, was carried out, and then, it was confirmed whether or not the nucleic acid can be extracted from the pseudo-tissue by means of measurement of absorbance in the wavelength of 260 nm. This pseudo-tissue was prepared as a replacement for a lymph node that has been excised from a living organism.

A homogenizing reagent that has the below described composition was prepared.

200 mM (pH 3.0) of glycin-HCl (Wako Pure Chemical Industries, Ltd.)

5 % of Brij 35 (Sigma)

0.05 % of KS-538 (Shin-Etsu Chemical Co., Ltd.)

The above described concentrations indicate the concentrations in the reagent.

As shown below, samples i and ii for measurement are prepared using a homogenizing reagent and/or an agarose solution.

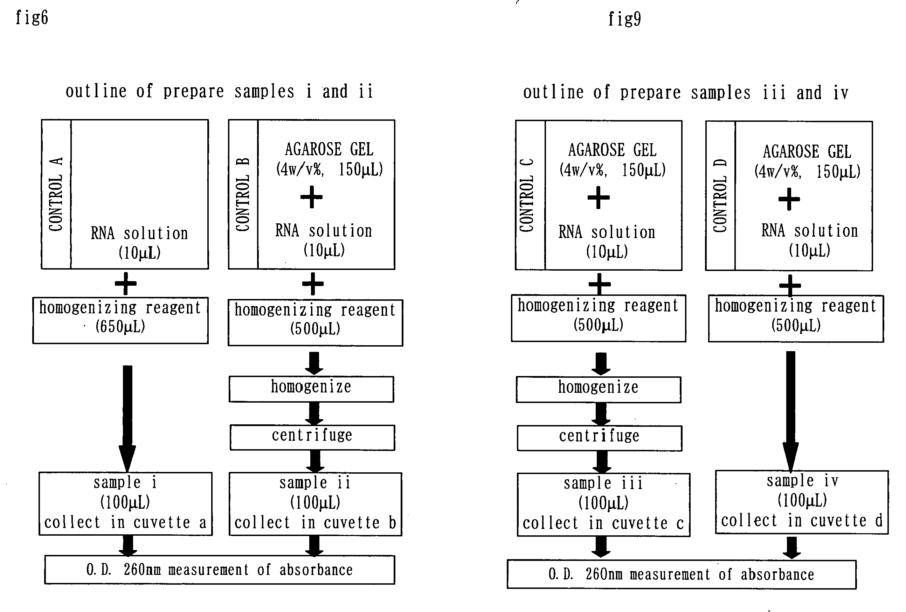

Fig 6 schematically shows the preparation of the samples for measurement that was carried out in Experiment 1.

### <Preparation of Sample i for Measurement>

First, a pipet where a pipet chip is mounted in a microtube was used so as to collect 10 µL of a solution that contains 4.6 µg of RNA that was extracted from the cells of a mouse (hereinafter referred to as an RNA solution) , and this is referred to as Control A. Furthermore, 650 µL of a homogenizing was added to control A, and this mixture was mixed through pipetting. 100 µL was collected from this mixture, and this is referred to as sample i for measurement, which was collected in a cuvette a.

### <Preparation of Sample ii for Measurement>

First, purified water was collected in a microtube. Furthermore, 6 µg of agarose (trade name: NuSieve GTC Agarose, made by CAMBREX Corporation) was added to the contents of the microtube as a gelling agent, and purified water was further added, so that the total volume became 150 µL. This microtube was heated in a thermostatic bath of which the temperature was set at approximately 70 °C, until the agarose completely dissolved in the purified water. When the agarose was completely dissolved in the purified water, the microtube was taken out from the thermostatic bath, and the temperature of the agarose solution was cooled at room temperature. The concentration of the agarose in this agarose solution was 4 w/v %. Immediately before the agarose solution was converted to a gel (when the temperature of the agarose solution became approximately 40 °C) , 10 µL of the RNA solution was added to the agarose solution. The agarose solution to which the RNA solution was added was mixed through pipetting, in order to prevent uneven distribution of RNA in the agarose solution. After the addition of the RNA solution, the agarose solution was further left at room temperature, so that the agarose solution was converted to a gel and became a support (agarose gel) holding RNA inside. The agarose gel that was contained within the microtube is referred to as Control B. Control B corresponds to a pseudo-tissue for quality control according to the present invention.

Fig 7 is a diagram schematically showing the pseudo-tissue that was prepared as described above, and the microtube that contains this pseudo-tissue. Microtube 2 is provided with a container portion 5 for containing a pseudo-tissue and a lid 6 for sealing the container portion, and container portion 5 contains an agarose gel 3 as a support and RNA 4 held within this agarose gel 3.

500 µL of a homogenizing reagent was added to 160 µL of Control B, and crushing through ten reciprocations of a pestle was carried out, and in addition, the mixture was centrifuged with approximately 2000 g for approximately 1 minute, so as to collect 100 µL of supernatant above the sediment, and this is referred to as sample ii for measurement which is contained in a cuvette b.

Sample ii for measurement was prepared assuming that a pseudo-tissue can be sufficiently homogenized during pre-processing, and thus, a uniform homogenate was obtained.

It is desirable for the homogenizing reagent, the microtube, the pipet chips, the purified water, the agarose and cuvettes a and b which are used to be free of RNase.

### <Measurement of absorbance>

A spectrophotometer (UV-2500PC, made by Shimadzu Corporation) was used to determine absorbance (O. D. 260 nm) of the respective samples for measurement which were collected in cuvettes a and b, and the concentration of RNA was calculated for each sample for measurement.

The results of determination are shown in the following Table 1. In addition, Fig 8 is a graph showing the concentration of RNA in Table 1.

The above described experiment was carried out three times, and the average value of these determined values is shown in the column "measured value of absorbance."

The concentration of RNA that is included in each sample for measurement and was calculated from the determined value of absorbance is shown in the column "concentration of RNA (µg/mL)."

The percentage of the relative value of sample ii for measurement when the concentration of RNA (or determined value of absorbance) of sample i for measurement is assumed to be 100 % is shown in the column "collection ratio relative to sample i for measurement."

**[Table 1]**

| | measured value of absorbance | RNA concentration (µg/mL) | collection ratio (%) |
|---|---|---|---|
| sample i (control A+homogenizing reagent) | 0.1772 | 7.1 | 100.0 |
| sample ii (control B+homogenizing reagent) | 0.1450 | 5.8 | 81.8 |

As can be seen from Table 1 and Fig 8, the measured value of the concentration of RNA of sample i for measurement was 7.1 µg/mL. This corresponds to the concentration of RNA in the RNA solution that was contained within the microtube at the time of the preparation of Control A.

The measured value of the concentration of RNA of sample ii for measurement was 5.8 µg/mL, and the collection ratio relative to sample i for measurement was 81.8 %.

As can be seen from the results of determination80 % or more of the nucleic acid could be detected, relative to sample i for measurement, by carrying out pre-processing on the support holding the nucleic acid. This shows that the nucleic acid was effectively collected from the support, by carrying out ten reciprocations of homogenization.

Accordingly, the measured value of the concentration of RNA of sample ii for measurement can be assumed to be the target value in the below described Experiment 2.

### (Experiment 2)

In Experiment 2, the concentration of the nucleic acid was calculated and compared with the target values that were calculated in Experiment 1, in the case where a pseudo-tissue was prepared from a nucleic acid and a support holding the nucleic acid, and a predetermined process, such as homogenization, was carried out, and then, the concentration of the nucleic acid was measured, as well as the case where the concentration of the nucleic acid without carrying out homogenization.

Samples iii and iv for measurement were prepared as shown in the below.

Fig 9 schematically shows the preparation of the samples for measurement that was carried out in the present experiment.

### <Preparation of Samples iii and iv for Measurement>

In the preparation of sample iii for measurement, first, 500 µL of a homogenizing reagent was added to Control C of the same lot as Control B in Experiment 1. Furthermore, the same process as that for the preparation of sample ii for measurement was carried out, and 100 µL of supernatant was collected in cuvette c.

In the preparation of sample iv for measurement, first, 500 µL of a homogenizing reagent was added to Control D of the same lot as Control B in Experiment 1. 100 µL of supernatant was collected from this mixture of Control D and the homogenizing reagent in cuvette d. Sample iv for measurement was prepared without carrying out homogenization.

### <Measurement of absorbance>

A spectrophotometer (UV-2500PC, made by Shimadzu Corporation) was used to determine absorbance (0. D. 260 nm) of each sample for measurement that was contained in cuvettes c and d, and the concentration of RNA included in each sample for measurement was calculated.

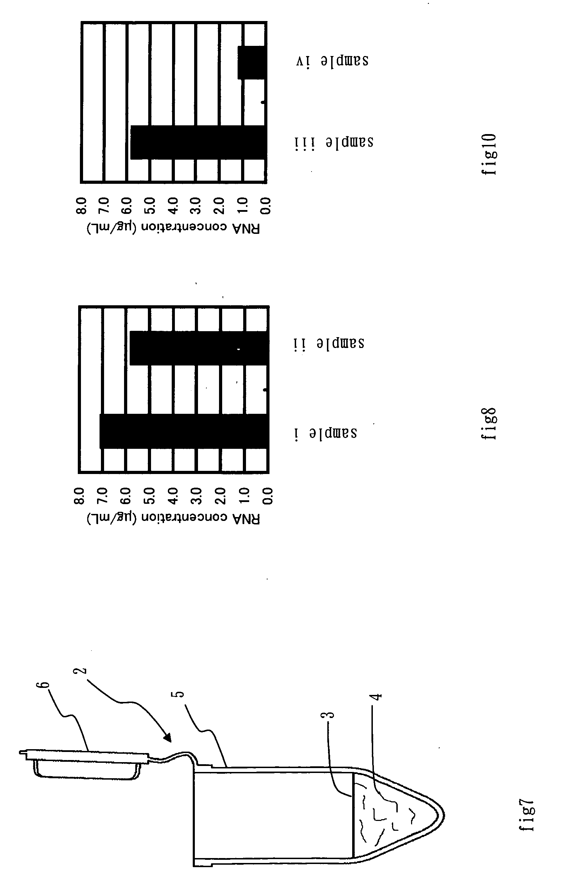

The results of determination are shown in the following Table 2. In addition, Fig 10 is a graph showing the concentration of RNA in Table 2.

The above described experiment was carried out three times, and the average of the determined values is shown in the column "measured value of absorbance" in Table 2.

The concentration of RNA that is included in each sample for measurement and was calculated from the measured value of absorbance is shown in the column "concentration of RNA (µg/mL)."

The percentage of relative values of samples iii and iv for measurement when the target values calculated in Experiment 1 are assumed to be 100 % is shown in the column "collection ratio relative to target value."

**[Table 2]**

| | measured value of absorbance | RNA concentration (µg/mL) | collection ratio (%) |
|---|---|---|---|
| sample iii (control C+homogenizing reagent) | 0.1450 | 5.8 | 100.0 |
| sample iv (control D+homogenizing reagent) | 0.0296 | 1.2 | 20.4 |

As can be seen from Table 2 and Fig 10, the measured value of the concentration of RNA of sample iii for measurement was 5.8 µg/mL. This value indicates a collection ratio of 100 % relative to the target value.

In addition, the measured value of the concentration of RNA of sample iii for measurement, which was measured without homogenization, was 1.2 µg/mL. This value indicates a collection ratio of 20.4 % relative to the target value.

It can be seen from the above described results of measurement that a nucleic acid cannot be effectively extracted from a pseudo-tissue without carrying out homogenization.

Accordingly, it can be confirmed whether or not homogenization is precisely carried out by comparing the target value and the measured value of the concentration of a nucleic acid in a pseudo-tissue.

It was confirmed in Experiments 1 and 2 that the pseudo-tissues (Controls B to D) which were prepared in the respective experiments were appropriate for the quality control of homogenization in gene examination.

### (Experiment 3)

In experiment 3, a pseudo-tissue made of cells that include a target nucleic acid and a support holding the cells that include the target nucleic acid, was used to measure the time to start amplification of CK19 mRNA that is included in the cells, and thereby, it was confirmed whether or not the nucleic acid could be extracted from the pseudo-tissue and amplified. This pseudo-tissue was prepared as a replacement for a lymph node that has been excised from a living organism. Here, the time when the turbidity reaches 0. 1 was calculated as the time to start amplification.

As described below, samples v, vi and vii for measurement were prepared using a homogenizing reagent and/or an agarose solution. The time to start amplification of sample v for measurement was used as a positive control in the present experiment, and the time to start amplification of sample vii for measurement was used as a negative control.

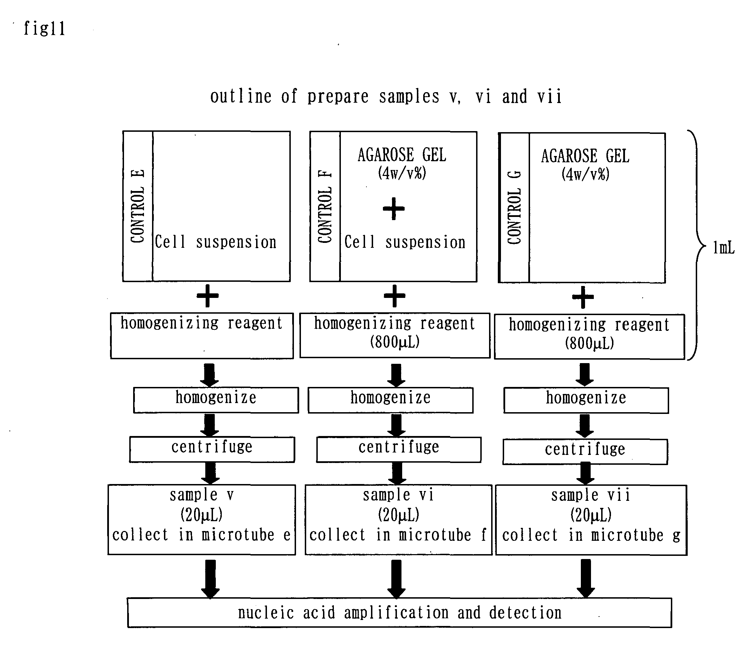

Fig 11 schematically shows the preparation of the samples for measurement that were carried out in the present experiment.

### <Preparation of Sample v for Measurement>

First, the number of cells originating from a human mammary cancer (MDA-MB-231; Dainippon Pharmaceutical Co., Ltd.) were counted through a microscope using a counting chamber (made by As One Corporation), and a cell suspension was prepared, so that 2 x 10⁶ cells were included in the solution. This cell suspension was contained in a microtube, and this is referred to as Control E. The homogenizing reagent that was used in Experiment 1 was added to Control E, so that the total became 1 ml. Crushing through 10 reciprocations of a pestle was carried out on the mixture of Control E and the homogenizing reagent. The obtained homogenate was centrifuged with approximately 2000 g for approximately 1 minute, so as to collect 20 µl of supernatant, and this is referred to as sample v for measurement which was collected in a microtube e.

### <Preparation of Sample vi for Measurement>

First, purified water was collected in a microtube. 8 µg of agarose (made by CAMBREX Corporation) was added to this as a gelling agent. This microtube was heated in a thermostatic bath of which the temperature was set at approximately 70 °C , until the agarose completely dissolved in the purified water. When the agarose was completely dissolved in the purified water, the microtube was taken out from the thermostatic bath, and the same cell suspension as that used at the time of preparation of sample v for measurement was added to the agarose solution. The mixture of the agarose solution and the cell suspension was mixed through pipetting, in order to prevent uneven distribution of the cells in the agarose solution. The temperature of this solution was lowered in a freezer at -20 °C, so as to convert the mixture to a gel, and this is referred to as Control F. Here, the total volume of the agarose solution and the cell suspension was adjusted to 200 µl. The concentration of agarose in the agarose solution was 4 w/v %. Control F corresponds to a pseudo-tissue for quality control according to the present invention.

After thawing Control F, 800 µl of a homogenizing reagent was added, and crushing through ten reciprocations of a pestle was carried out. The obtained homogenate was centrifuged with approximately 2000 g for approximately 1 minute, so as to collect 20 µl of supernatant, and this is referred to as sample vi for measurement which was collected in a microtube f.

### <Preparation of Sample vii for Measurement>

First, Control G was prepared. Control G was prepared in the same manner as Control F, except that no cell suspension was used in Control G.

800 µl of a homogenizing reagent was added to Control G, and crushing through 10 reciprocations of a pestle was carried out. The obtained homogenate was centrifuged with approximately 2000 g for approximately 1 minute, so as to collect 20 µl of supernatant, and this is referred to as sample vii for measurement which was collected in a microtube g.

### <Measurement of Time to Start Amplification>

180 µl of a homogenizing reagent was added to microtubes e, f and g, respectively, so that samples v, vi and vii for measurement were diluted so that the total volume increased by ten.

A gene amplification detecting device GD-100 (made by Sysmex Corporation) was used to amplify cDNA that corresponds to CK19 mRNA in each of the diluted samples for measurement, and a change, such that the turbidity increases together with the amplification, was monitored in real time.

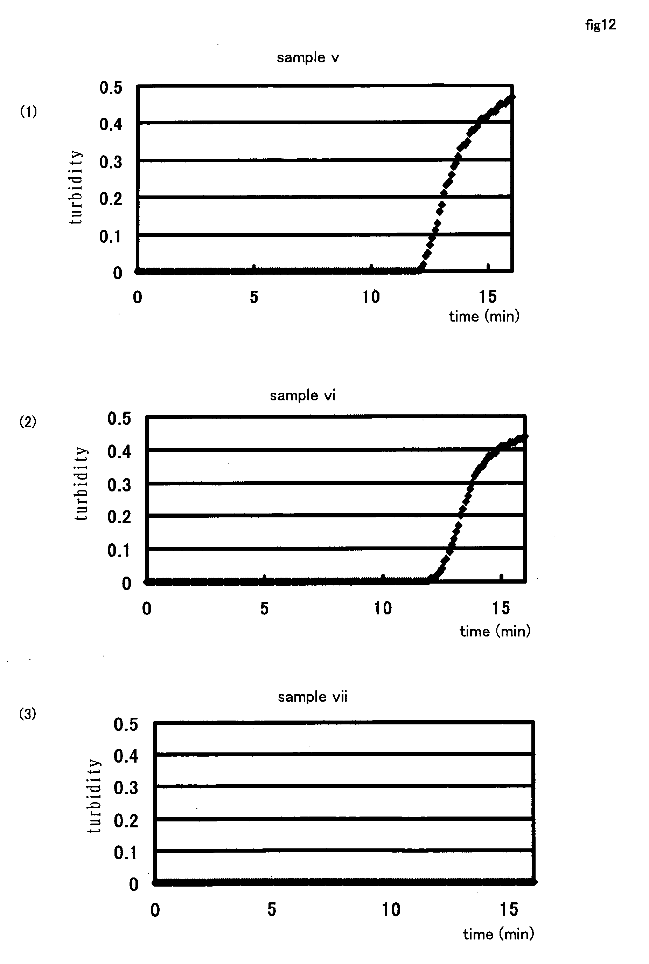

The results of the measurement are shown in Figs 12 (1) to 12 (3) .

Figs 12 (1) , 12 (2) and 12 (3) are graphs showing the time to start amplification of samples v, vi and vii for measurement, respectively.

The time to start amplification of sample v for measurement, which is a positive control, was 12.7 minutes, and the time to start amplification of sample vi for measurement was 12.9 minutes. In addition, the start of amplification of sample vii for measurement, which is a negative control, was not observable.

It can be confirmed from the above described results of measurement that the value for the time to start amplification of a sample for measurement that is obtained from a pseudo-tissue is very close to that of the time to start amplification of sample v for measurement, which is a positive control, in the case where homogenization of the pseudo-tissue is precisely carried out, and it can be judged that it possible to use this pseudo-tissue as a material for quality control.

Though in the present specification, a case where pre-processing is carried out on a tissue that has been excised from a living organism and a nucleic acid that is included in the excised tissue is detected, and thereby, the existence of an ailment can be assumed is described, it is also possible to assume the existence of an ailment by measuring the revealed amount or activity of a particular protein that is included in the excised tissue. In this case also, pre-processing of the excised tissue is necessary, and thus, quality control of the pre-processing can be carried out by detecting a protein using a pseudo-tissue made of cells and a support holding the cells. A protein that is included in the pre-processed pseudo-tissue can be detected through measurement of absorbance, or analysis of the revealed amount or activity of the protein.

## Claims

1. A pseudo-tissue for quality control, **characterized by** comprising:
a nucleic acid component selected from the group consisting of a nucleic acid and a cell including a nucleic acid; and
a gel for holding nucleic acid component.

2. The pseudo-tissue according to claim 1, wherein said nucleic acid is RNA.

3. The pseudo-tissue according to claim 1 or 2, wherein said RNA is mRNA.

4. The pseudo-tissue according to any one of claims 1 to 3, wherein said cells include oncocytes.

5. The pseudo-tissue according to any one of claims 1 to 4, wherein said gel comprises a gelling agent and a solvent.

6. The pseudo-tissue according to any one of claims 1 to 5, wherein said gel fluidizes when heated.

7. The pseudo-tissue according to any one of claims 1 to 6, wherein said gel includes polymer selected from the group consisting of natural polymers and synthesized polymers.

8. The pseudo-tissue according to any one of claims 1 to 7, wherein said gel includes polymer selected from the group consisting of agar, agarose, carageenan, alginic acid, alginates, pectin, collagen, gelatin, gluten, polyvinyl alcohol, polyethylene glycol and polyacrylamide.

9. A quality control method, **characterized by** comprising the steps of:
measuring the concentration of a nucleic acid in a sample that is obtained by carrying out a nucleic acid extracting process including homogenization on a pseudo-tissue having a predetermined nucleic acid concentration, the pseudo-tissue comprising a nucleic acid component and a support for holding the nucleic acid component, the nucleic acid component selected from the group consisting of a nucleic acid and a cell including a nucleic acid; and
comparing the measured concentration with the predetermined nucleic acid concentration so as to determine whether or not said nucleic acid extracting process was appropriately carried out.

10. A quality control method, **characterized by** comprising the steps of:
amplifying a target nucleic acid in a sample that is obtained by carrying out a nucleic acid extracting process including homogenization on a pseudo-tissue having a predetermined target nucleic acid concentration, the pseudo-tissue comprising a nucleic acid component and a support for holding the nucleic acid component, the nucleic acid component selected from the group consisting of a target nucleic acid and a cell including a target nucleic acid;
measuring the concentration of the target nucleic acid originating from said pseudo-tissue by detecting the amplified target nucleic acid; and
comparing the measured concentration with the predetermined nucleic acid concentration so as to determine whether or not the steps of amplifying and measuring were appropriately carried out.

11. The method according to claim 9 or 10, wherein said support is a gel.

12. The method according to any one of claims 9 to 11, wherein said nucleic acid is RNA.

13. The method according to any one of claims 9 to 12, wherein said cells include oncocytes.

14. The method according to any one of claims 9 to 13, wherein said support includes polymer selected from the group consisting of agar, agarose, carageenan, alginic acid, alginates, pectin, collagen, gelatin, gluten, polyvinyl alcohol, polyethylene glycol and polyacrylamide.

15. The method according to claim 10, wherein said target nucleic acid is at least one selected from the group consisting of a gene which codes a portion of a tumor marker, a gene which codes the entirety of a tumor marker, mRNA which corresponds to each of said genes, and a partial alignment of these.

16. The method according to claim 10 or 19, wherein said tumor marker is cytokeratin.
